# EUROPÄISCHE PATENTSCHRIFT

(11) **EP 0 912 142 B1**
(45) Veröffentlichungstag und Bekanntmachung des Hinweises auf die Patenterteilung: **16.10.2002**
(21) Anmeldenummer: 97931647.8
(22) Anmeldetag: 24.06.1997
(51) Int. Cl.: A61B 18/12

(54) **ELEKTRODE MIT GLATTER, DREHBARER OBERFLÄCHE ZUR HF-CHIRURGIE**
SMOOTH-SURFACE ROTARY ELECTRODE FOR HF SURGERY
ELECTRODE ROTATIVE A SURFACE LISSE POUR CHIRURGIE PAR HAUTE FREQUENCE

(30) Priorität: 24.06.1996 DE 19625242
(43) Veröffentlichungstag der Anmeldung: 06.05.1999
(73) Patentinhaber: Karl Storz GmbH & Co. KG, 78532 Tuttlingen (DE)
(72) Erfinder: IRION, Klaus, D-78576 Liptingen (DE); SOLINGEN, Simon, Los Angeles, CA 90077 (US)
(74) Vertreter: Münich, Wilhelm, Dr.
(86) Internationale Anmeldenummer: DE9701310
(87) Internationale Veröffentlichungsnummer: WO97049346

(56) Entgegenhaltungen:
- DE-A- 2 222 820
- DE-A- 4 242 126
- DE-A- 19 514 552
- US-A- 5 395 363
- US-A- 5 549 605
- US-A- 5 599 349
- US-A- 5 634 924

## Beschreibung

Die Erfindung bezieht sich auf eine Elektrode zur HF-Chirurgie und insbesondere zum Vaporisieren von Gewebe gemäß dem Oberbegriff des Patentanspruchs 1.

Für eine Reihe von Behandlungsaufgaben werden Elektroden benötigt, die sich rollend über das Gewebe bewegen lassen, um einen gleichmäßigen Behandlungserfolg über eine größere Fläche zu erzielen:

Beispielsweise ist eine Elektrode zur HF-Chirurgie, von der bei der Formulierung des Oberbegriffs des Patentanspruchs 1 ausgegangen worden ist, aus der DE 42 42 126 C1 bekannt. Diese bekannte Elektrode weist einen Rotationskörper auf, der an seiner Oberfläche in das Endometrium einsinkende Erhebungen aufweist, die insbesondere als Stacheln ausgebildet sind. Durch das Einsinken der an dem Rotationskörper vorgesehenen Erhebungen soll sich die größte Stromdichte in der Tiefe des Gewebes und nicht an der Oberfläche ergeben.

Weitgehend ähnliche Elektroden sind aus der US-PS 5,395,363 oder der DE-OS 22 22 820 bekannt.

In einer Reihe von Fällen ist es jedoch nicht erwünscht, wenn der Elektrodenkörper in das Gewebe eindringt. Deshalb ist bei einer weiteren gattungsgemäßen Elektrode zur HF-Chirurgie der Elektrodenkörper nicht mit Erhebungen, sondern mit Vertiefungen ausgestattet, aufgrund derer der Elektrodenkörper zwar nicht in das Gewebe einsinkt, durch die aber eine spezielle Stromverteilung erreicht wird. Hierzu wird auf das DE 295 11 618 U1 verwiesen.

Aber auch bei dieser Ausbildung ergeben sich in der Praxis Nachteile, wie beispielsweise eine erhöhte Verletzungsgefahr aufgrund der Rücksprünge bzw. Vertiefungen, in denen sich Gewebeteile "verfangen" können.

Der Erfindung liegt die Aufgabe zugrunde, eine Elektrode zur HF-Chirurgie und insbesondere zum Vaporisieren von Gewebe anzugeben, die einen über das Gewebe rollenden Elektrodenkörper aufweist, und bei der man eine bestimmte Stromdichte-Verteilung erhält, ohne daß die mit der Verwendung von Erhöhungen bzw. Vertiefungen verbundenen Nachteile auftreten.

Eine erfindungsgemäße Lösung dieser Aufgabe ist im Patentanspruch 1 angegeben. Weiterbildungen der Erfindung sind Gegenstand der Ansprüche 2 bis 5. In den Ansprüchen 14 bis 19 ist ein Verfahren zur Herstellung einer erfindungsgemäßen Elektrode angegeben.

Die Erfindung bricht mit der bei gattungsgemäßen Elektroden, die mindestens einen abrollenden Elektrodenkörper aufweisen, offensichtlich allgemeinen Anschauungen, daß lediglich ein Elektrodenkörper, bei dem Erhöhungen oder Vertiefungen vorgesehen sind, die gewünschte Verteilung des Stromflusses und damit den gewünschten Behandlungserfolg sicherstellen kann.

Durch die Erfindung wird eine Elektrode mit mindestens einem Elektrodenkörper, der über das Gewebe abrollt, geschaffen, dessen Oberfläche, die mit dem Gewebe in Kontakt kommt glatt ist. In der glatten Oberfläche sind Bereiche niedriger elektrischer Leitfähigkeit oder isolierende Bereiche und eine Vielzahl von in der Oberfläche nicht zusammenhängender Bereiche mit hoher elektrischer Leitfähigkeit vorgesehen. Die Bereiche hoher elektrischer Leitfähigkeit können dabei zu einer oder mehreren Gruppen zusammengefaßt werden, wobei die Bereiche jeder Gruppe im Elektrodenkörper oder außerhalb des Elektrodenkörpers elektrisch leitend miteinander verbunden sind.

Die Verbindung kann dabei derart erfolgen, daß sämtliche Bereiche miteinander verbunden sind, - also nur eine Gruppe vorhanden ist -,so daß man eine monopolar arbeitende Elektrode erhält (Anspruch 3).

Ferner ist es möglich, daß die elektrisch leitenden Bereiche zu wenigstens zwei im Elektrodenkörper jeweils miteinander verbundenen Gruppen zusammengefaßt sind, so daß die erfindungsgemäße aufgebaute Elektrode als bipolare Elektrode eingesetzt werden kann (Anspruch 4).

Bevorzugt bestehen gemäß Anspruch 2 die Bereiche niedriger elektrischer Leitfähigkeit aus einem elektrisch isolierenden Material.

Dadurch, daß die einzelnen Bereiche einen definierten Oberflächen-Anteil haben (Anspruch 5), kann eine definierte Stromdichte-Verteilung eingestellt werden.

Im Anspruch 6 ist eine Weiterbildung gekennzeichnet, gemäß der der Elektrodenkörper einen elektrisch gut leitenden zentralen Bereich aufweist, der hin zu der glatten Oberfläche, die mit dem Gewebe in Kontakt kommt, Vorsprünge hat. Die Bereiche zwischen den Vorsprüngen sind mit einem Material mit geringer elektrischer Leitfähigkeit bzw. mit isolierenden Eigenschaften ausgefüllt, so daß sich die erfindungsgemäß eingesetzte glatte Oberfläche ergibt. Die Oberflächen-Bereiche mit guter elektrischer Leitfähigkeit und die Oberflächen-Bereiche mit geringer elektrischer Leitfähigkeit bzw. mit isolierenden Eigenschaften können statistisch verteilt (Anspruch 7) oder regelmäßig angeordnet sein (Anspruch 8). Welche der beiden Möglichkeiten gewählt wird, hängt dabei von der jeweiligen medizinischen Indikation ab.

Im Falle regelmäßig angeordneter Vorsprünge kann der zentrale Bereich zur glatten Oberfläche hin eine Längsund/oder Querrillung und/oder Erhebungen aufweisen, je nach dem, welche der verschiedenen Möglichkeiten medizinisch indiziert ist und/oder herstellungstechnisch am einfachsten herzustellen ist.

In jedem Falle ist es jedoch bevorzugt, wenn die gesamte Flächenausdehnung der Bereiche mit guter elektrischer Leitfähigkeit kleiner als die der Bereiche mit schlechter elektrischer Leitfähigkeit ist, da sich hierdurch eine für die Vaporisation besonders günstige Stromdichte-Verteilung einstellt.

Den erfindungsgemäßen Grundgedanken, einen Elektrodenkörper mit glatter Oberfläche zu verwenden, in der lediglich ein Teil der Oberfläche zum Stromfluß beiträgt, kann auf die verschiedensten Elektroden angewendet werden:

So kann der Elektrodenkörper die Form eines Zylinders (Anspruch 11) oder einer Kugel (Anspruch 12) haben, die drehbar gelagert sind, so daß die Elektrode rollend über das Gewebe bewegt werden kann bei Elektrodenkörpern angewandt werden, die die Form einer Platte oder eines Bandes haben (Anspruch 14) und die wenigstens in einer Richtung gebogen sind.

Die Sicherheit für den Patienten wird durch die Ausbildung gemäß Anspruch 13 erhöht, gemäß der das Material mit niedriger elektricher Leitfähigkeit bzw. mit isolierenden Eigenschaften über einen Formschluß mit dem zentralen Elektrodenkörper verbunden ist, da hierdurch die Gefahr verringert wird, daß Materialabplatzungen zu Ablagerungen im Körper führen.

In den Ansprüchen 14 f. sind Verfahren zur Herstellung einer erfindungsgemäßen Elektrode beschrieben.

Gemäß Anspruch 14, bei dem ein Verfahren zur Herstellung einer Elektrode beschrieben wird, wird zunächst ein wenigstens einen elektrisch leitenden Bereich aufweisender Elektrodenkörper hergestellt, in den in dem Bereich der Oberfläche, die mit dem zu behandelnden Gewebe in Kontakt kommt, Ausnehmungen eingebracht werden. Die Ausnehmungen werden mit einem Material schlechter elektrischer Leitfähigkeit ausgefüllt, so daß eine glatte Oberfläche entsteht. Dabei kann insbesondere ein Beschichtungsprozeß (Anspruch 15) oder ein Aufdampfvorgang (Anspruch 16) verwendet werden. Nach dem Ausfüllen der Ausnehmungen kann die Elektrode zumindest in dem Teil der Oberfläche, der mit dem zu behandelnden Gewebe in Kontakt kommt, beispielsweise spanabhebend bearbeitet werden (Ansprüche 17 bzw. 18).

Hierdurch erhält man eine definierte und entsprechend dem gewählten Bearbeitungsvorgang glatte Oberfläche.

Im Anspruch 19 ist angegeben, daß in den Ausnehmungen in dem Elektrodenkörper Hinterschneidungen etc. vorgesehen sind, so daß das Material mit geringer elektrischer Leitfähigkeit über einen Formschluß an dem Elektrodenkörper haftet. Hierdurch wird - wie bereits ausgeführt - die Sicherheit für den Patienten weiter erhöht. In jedem Falle erhält man durch die erfindungsgemäße Ausbildung des Elektrodenkörpers überraschende Vorteile sowohl gegenüber bekannten abrollenden Elektrodenkörpern mit Erhöhungen oder Vertiefungen als auch gegenüber Elektrodenkörpern, die starr angeordnet sind, und die - hierzu wird auf die US-PS 3,460,539 verwiesen - eine glatte Oberfläche mit in der Oberfläche verteilten Bereichen unterschiedlicher Leitfähigkeit aufweisen: Gegenüber diesem Stand der Technik ergibt sich durch das Abrollen der glatten Oberfläche, in der Bereiche unterschiedlicher Leitfähigkeit vorhanden sind, auf dem Gewebe eine besonders gleichmäßige und effektive Beaufschlagung des Gewebes mit HF-Strom.

Die Erfindung wird nachstehend anhand eines Ausführungsbeispiels unter Bezugnahme auf die Zeichnung näher beschrieben, in der zeigen:
- Fig. 1a: den Elektrodenkörper eines ersten Ausführungsbeispiels der Erfindung in einem noch unvollständigen zustand,
- Fig. 1b: die vollständige Elektrode bei diesem Ausführungsbeispiel, und
- Fig. 2: die Elektrode eines zweiten Ausführungsbeispiels der Erfindung.

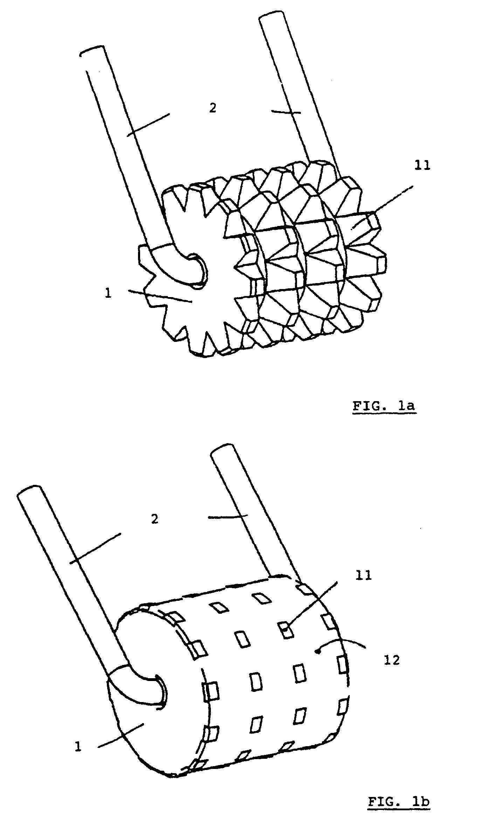

In Fig. 1 ist ein Elektrodenkörper 1 einer monopolar arbeitenden HF-Elektrode zur HF-Chirurgie und insbesondere zum Vaporisieren von Gewebe dargestellt. Der Elektrodenkörper 1 weist zwei Zuleitungen 2 auf, über die er mit einem Pol einer HF-Versorgungseinheit (nicht dargestellt) verbunden ist. Bei dem gezeigten Ausführungsbeispiel ist der Elektrodenkörper 1 ein drehbar gelagerter Zylinder, der an seiner Oberfläche Erhebungen 11 aufweist. Der Elektrodenkörper besteht aus einem elektrisch leitenden Material, wie es für die Herstellung von HF-Elektroden allgemein verwendet wird.

Nicht dargestellt ist in den Figuren das Führungselement, das in an sich bekannter Weise, beispielsweise in der aus der US-PS 5,395,363 oder in einer aus der Endoskopie bekannten Weise ausgebildet sein kann.

Fig. 1a zeigt eine noch unvollständige Elektrode bei der die Ausnehmungen zwischen den Elektroden noch nicht mit einem elektrisch isolierenden Material ausgefüllt sind.

Fig. 1b zeigt die fertige Elektrode, bei der die Ausnehmungen zwischen den Erhebungen 11 mit einem elektrisch isolierenden Material 12 derart ausgefüllt sind, daß die Oberfläche des Elektrodenkörpers 1 eine glatte Zylinderfläche ist.

Die Elektrode kann damit rollend über das Gewebe bewegt werden, ohne daß sie in das Gewebe einsinkt oder daß die Gefahr von Verletzungen bzw. von Verhakungen von Gewebeteilen in Ausnehmungen besteht.

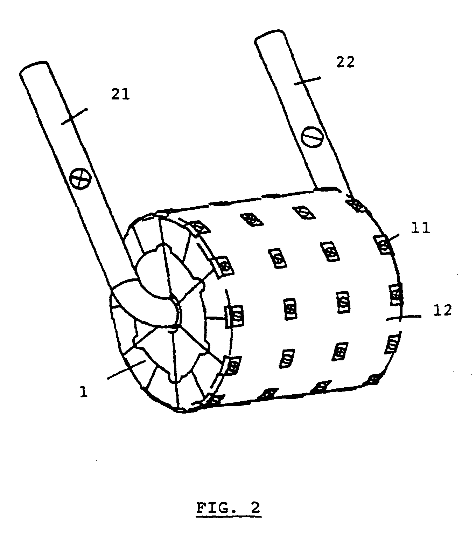

Fig. 2 zeigt ein Ausführungsbeispiel der Erfindung, bei dem die Elektrode eine bipolare Elektrode ist. Zur Realisierung einer bipolaren Elektrode ist die Zuleitung 21 mit dem einen Pol einer nicht dargestellten HF-Versorgungseinheit verbunden, während die Zuleitung 22 mit dem anderen Pol der HF-Versorgungseinheit verbunden ist. Entsprechend dieser Ausbildung sind in der Oberfläche des Elektrodenkörpers 1 zwei Gruppen von Erhebungen 11 vorgesehen, von denen die eine Gruppe mit der Zuleitung 21 und die andere Gruppe mit der Zuleitung 22 elektrisch verbunden ist. Dies ist durch Minus- bzw. Plus-Zeichen in den einzelnen Erhebungen 11 angedeutet. Der Raum zwischen den Erhebungen 11 ist wiederum mit einem elektrisch isolierenden Material 12 ausgefüllt.

Bewegt man nun den Elektrodenkörper 1 rollend über das zu behandelnde Gewebe, so ergibt sich ein Stromfluß über das Gewebe zwischen den mit der Zuleitung 21 und den mit der Zuleitung 22 verbundenen Erhebungen 11.

Die Erfindung ist vorstehend anhand von Ausführungsbeispielen ohne Beschränkung des allgemeinen Erfindungsgedankens beschrieben worden.

## Patentansprüche

1. Elektrode zur HF-Chirurgie und insbesondere zum Vaporisieren von Gewebe, mit einem Führungselement für mindestens einen Elektrodenkörper (1), der über wenigstens eine Zuleitung (2) mit einer HF-Versorgungseinheit verbunden ist und wenigstens teilweise mit dem zu behandelnden Gewebe in Kontakt kommt, und der eine Abrollachse aufweist, die senkrecht zur Längsachse des Führungselements verläuft, **dadurch gekennzeichnet, daß** jeder Elektrodenkörper (1) in dem Bereich, der mit dem Gewebe in Kontakt kommt, eine glatte Oberfläche aufweist, in der Bereiche niedriger elektrischer Leitfähigkeit und eine Vielzahl von in der Oberfläche nicht zusammenhängender Bereiche mit hoher elektrischer Leitfähigkeit vorgesehen sind, und
daß jeweils bestimmte oder alle Bereiche hoher elektrischer Leitfähigkeit im Inneren des Elektrodenkörpers (1) oder außerhalb des Elektrodenkörpers (1) miteinander elektrisch verbunden sind.

2. Elektrode nach Anspruch 1,
**dadurch gekennzeichnet, daß** die Bereiche niedriger elektrischer Leitfähigkeit elektrisch isolierende Bereiche sind.

3. Elektrode nach Anspruch 1 oder 2,
**dadurch gekennzeichnet, daß** die elektrisch leitenden Bereiche im Elektrodenkörper (1) mit einer Zuleitung (2)verbunden sind, so daß die Elektrode eine monpolare Elektrode ist.

4. Elektrode nach Anspruch 1 oder 2,
**dadurch gekennzeichnet, daß** die elektrisch leitenden Bereiche zu wenigstens zwei im Elektrodenkörper (1) miteinander elektrisch verbundener Gruppen zusammengefaßt sind, so daß die Elektrode eine bipolare Elektrode ist.

5. Elektrode nach einem der Ansprüche 1 bis 4,
**dadurch gekennzeichnet, daß** die einzelnen Bereiche einen definierten Oberflächen-Anteil haben.

6. Elektrode nach einem der Ansprüche 1 bis 5,
**dadurch gekennzeichnet, daß** der Elektrodenkörper (1) einen elektrisch gut leitenden zentralen Bereich aufweist, der hin zu der glatten Oberfläche, die mit dem Gewebe in Kontakt kommt, Vorsprünge hat, und daß die Bereiche zwischen den Vorsprüngen mit einem Material (12) mit geringer elektrischer Leitfähigkeit bzw. mit isolierenden Eigenschaften ausgefüllt sind.

7. Elektrode nach Anspruch 6,
**dadurch gekennzeichnet, daß** die Oberflächen-Bereiche mit guter elektrischer Leitfähigkeit und die Oberflächen-Bereiche mit geringer elektrischer Leitfähigkeit bzw. mit isolierenden Eigenschaften statistisch verteilt sind.

8. Elektrode nach Anspruch 6,
**dadurch gekennzeichnet, daß** die Vorsprünge regelmäßig angeordnet sind.

9. Elektrode nach Anspruch 8,
**dadurch gekennzeichnet, daß** der zentrale Bereich zur glatten Oberfläche hin eine Längs- und/oder Querrillung und/oder Erhebungen (11) aufweist.

10. Elektrode nach einem der Ansprüche 1 bis 9,
**dadurch gekennzeichnet, daß** die gesamte Flächenausdehnung der Bereiche mit guter elektrischer Leitfähigkeit kleiner als die der Bereiche mit schlechter Leitfähigkeit ist.

11. Elektrode nach einem der Ansprüche 1 bis 10,
**dadurch gekennzeichnet, daß** der Elektrodenkörper (1) die Form eines Zylinders hat, der drehbar gelagert ist.

12. Elektrode nach einem der Ansprüche 1 bis 10,
**dadurch gekennzeichnet, daß** der Elektrodenkörper (1) die Form einer Kugel hat, die drehbar gelagert ist, so daß die Elektrode rollend über das Gewebe bewegt werden kann.

13. Elektrode nach einem der Ansprüche 1 bis 14,
**dadurch gekennzeichnet, daß** das Material (12) mit niedriger elektrischer Leitfähigkeit bzw. mit isolierenden Eigenschaften über einen Formschluß mit dem zentralen Elektrodenbereich verbunden ist.

14. Verfahren zur Herstellung einer Elektrode nach einem der Ansprüche 1 bis 13 mit folgenden Schritten: - zunächst wird ein wenigstens einen elektrisch leitenden Bereich aufweisender Elektrodenkörper (1) hergestellt, - in den Bereich der Oberfläche des Elektrodenkörpers (1), der mit dem zu behandelnden Gewebe in Kontakt kommt, werden Ausnehmungen (11) eingebracht, - die Ausnehmungen (11) werden mit einem Material (12) schlechter elektrischer Leitfähigkeit ausgefüllt, so daß eine glatte Oberfläche entsteht.

15. Verfahren nach Anspruch 14,
**dadurch gekennzeichnet, daß** die Ausnehmungen (11) mit einem Beschichtungsprozeß ausgefüllt werden.

16. Verfahren nach Anspruch 15,
**dadurch gekennzeichnet, daß** es sich bei dem Beschichtungsprozeß um einen Aufdampfvorgang handelt.

17. Verfahren nach einem der Ansprüche 14 bis 16,
**dadurch gekennzeichnet, daß** nach dem Ausfüllen der Ausnehmungen (11) zumindest der Teil der Oberfläche, der mit dem zu behandelnden Gewebe in Kontakt kommt, bearbeitet wird.

18. Verfahren nach Anspruch 17,
**dadurch gekennzeichnet, daß** der Bearbeitungsvorgang ein spanabhebender Bearbeitungsvorgang ist.

19. Verfahren nach einem,der Ansprüche 14 bis 18,
**dadurch gekennzeichnet, daß** in den Ausnehmungen (11) Hinterschneidungen etc. vorgesehen sind, so daß das Material (12) geringer elektrischer Leitfähigkeit über einen Formschluß an dem Elektrodenkörper (1) haftet.

## Claims

1. Electrode for HF surgery and specifically for evaporating tissue, comprising a guiding element for at least one electrode body (1) that is connected via at least one feed line (2) to an HF supply unit and comes into contact with the tissue to be treated, at least partly, and that presents a rolling axis extending orthogonally on the longitudinal axis of said guiding element,
**characterised in that** each electrode body (1) has a smooth surface in the area coming into contact with the tissue, in which surface areas of low electrical conductivity and a plurality of areas of high electrical conductivity are provided, which are not coherent in the surface, and
that certain or all areas of a high electrical conductivity are electrically interconnected inside said electrode body (1) or outside said electrode body (1).

2. Electrode according to Claim 1,
**characterised in that** said areas of low electrical conductivity are electrically insulating areas.

3. Electrode according to Claim 1 or 2,
**characterised in that** said electrically conductive areas in said electrode body (1) are connected to a feed line (2) such that the electrode is a monopole electrode.

4. Electrode according to Claim 1 or 2,
**characterised in that** said electrically conductive areas are combined to form at least two groups electrically connected to each other in said electrode body (1) so that the electrode is a bipolar electrode.

5. Electrode according to any of the Claims 1 to 4,
**characterised in that** the individual areas correspond to a defined surface fraction.

6. Electrode according to any of the Claims 1 to 5,
**characterised in that** said electrode body (1) comprises an electrically well conducting central region having projections towards the smooth surface coming into contact with the tissue, and that the areas between said projections are filled with a material (12) of low electrical conductivity or with insulating properties, respectively.

7. Electrode according to Claim 6,
**characterised in that** the surface areas of good electrical conductivity and the surface areas of low electrical conductivity or with insulating properties, respectively, are distributed in a statistical pattern.

8. Electrode according to Claim 6,
**characterised in that** said projections are disposed in a regular array.

9. Electrode according to Claim 8,
**characterised in that** said central region presents longitudinal and/or transverse flutes and/or projections (11) in a direction towards the smooth surface.

10. Electrode according to any of the Claims 1 to 9,
**characterised in that** the entire two-dimensional extension of said areas of good electrical conductivity is smaller than the extension of the areas of low conductivity.

11. Electrode according to any of the Claims 1 to 10,
**characterised in that** said electrode body (1) has the shape of a cylinder supported for rotation.

12. Electrode according to any of the Claims 1 to 10,
**characterised in that** said electrode body (1) has the shape of a sphere supported for rotation so that the electrode may be moved in a rolling movement over the tissue.

13. Electrode according to any of the Claims 1 to 14,
**characterised in that** said material (12) of low electrical conductivity or with insulating properties, respectively, is connected to said central electrode region via a positive engagement.

14. Method for manufacturing an electrode according to any of the Claims 1 to 13, comprising the following steps: - initially, an electrode body (1) is produces that comprises at least one electrically conductive area - then recesses (1) are formed in that area of the surface of the electrode body (1), which comes into contact with the tissue to be treated - and said recesses (11) are filled with a material (12) of low electrical conductivity so that a smooth surface is created.

15. Method according to Claim 14,
**characterised in that** said recesses (11) are filled in a coating process.

16. Method according to Claim 15,
**characterised in that** said coating process is a vacuum metallization process.

17. Method according to any of the Claims 14 to 16,
**characterised in that**, after said recesses (11) have been filled, at least that part of the surface, which comes into contact with the tissue to be treated, is processed.

18. Method according to Claim 17,
**characterised in that** said processing operation is a machining operation.

19. Method according to any of the Claims 14 to 18,
**characterised in that** undercuts etc. are provided in said recesses (11) so that said material (12) of low electrical conductivity will adhere to said electrode body (1) via a positive engagement.

## Revendications

1. Électrode pour chirurgie par haute fréquence et en particulier à vaporiser, comprenant un élément de guidage pour au moins un corps d'électrode (1), qui est relié, via au moins un conducteur d'amenée (2), à une unité d'alimentation HF et vient en contact avec le tissu à traiter, au moins en partie, et qui comprend un axe de roulement, qui s'étend en sens orthogonal sur l'axe longitudinal dudit élément de guidage,
**caractérisée en ce que** chaque corps d'électrode (1) a une surface lisse dans la zone venant en contact avec le tissu, dans laquelle des zones à basse conductibilité électrique et une pluralité de zones à haute conductibilité électrique sont formées, qui ne sont pas cohérente dans la surface, et
**en ce que** certaines ou toutes les zones à haute conductibilité électrique sont électriquement reliées l'une à l'autre à l'intérieur dudit corps d'électrode (1) ou à l'extérieur dudit corps d'électrode (1).

2. Électrode selon la revendication 1,
**caractérisée en ce que** lesdites zones à basse conductibilité électrique sont des zones électriquement isolantes.

3. Électrode selon la revendication 1 ou 2,
**caractérisée en ce que** lesdites zones électriquement conductibles dans ledit corps d'électrode (1) sont reliées à un conducteur d'amenée (2) de façon, que l'électrode constitue une électrode mono polaire

4. Électrode selon la revendication 1 ou 2,
**caractérisée en ce que** lesdites zones électriquement conductibles sont combinées de façon à former au moins deux groupes électriquement reliés l'un à l'autre dans ledit corps d'électrode (1), d'une manière, que l'électrode constitue une électrode bipolaire.

5. Électrode selon une quelconque des revendication 1 à 4,
**caractérisée en ce que** les zones individuelles correspondent à une partie de surface définie.

6. Électrode selon une quelconque des revendication 1 à 5,
**caractérisée en ce que** ledit corps d'électrode (1) comprend une zone centrale à bonne conductibilité électrique, ayant des bosses vers la surface lisse venant en contact avec le tissu, et **en ce que** les zones entre lesdites bosses sont remplies d'un matériau (12) basse conductibilité électrique ou respectivement aux propriétés isolantes.

7. Électrode selon la revendication 6,
**caractérisée en ce que** les zones superficielles à bonne conductibilité électrique et les zones superficielles à basse conductibilité électrique ou respectivement aux propriétés isolantes sont distribuées dans une structure statistique.

8. Électrode selon la revendication 6,
**caractérisée en ce que** lesdites bosses sont disposées en un arrangement régulier.

9. Électrode selon la revendication 8,
**caractérisée en ce que** ladite zone centrale présente des flûtes et/ou bosses (11) longitudinales et/ou transversales en un sens vers la surface lisse.

10. Électrode selon une quelconque des revendication 1 à 9,
**caractérisée en ce que** l'entière étendue bidimensionnelle desdites zones à bonne conductibilité électrique est plus petite que l'étendue des zones à basse conductibilité.

11. Électrode selon une quelconque des revendication 1 à 10,
**caractérisée en ce que** ledit corps d'électrode (1) a la forme d'un cylindre logé de façon rotative.

12. Électrode selon une quelconque des revendication 1 à 10,
**caractérisée en ce que** ledit corps d'électrode (1) a la forme d'une sphère logée de façon rotative et d'une manière, qu'on puisse passer l'électrode par un mouvement roulant sur le tissu.

13. Électrode selon une quelconque des revendication 1 à 14,
**caractérisée en ce que** ledit matériau (12) à basse conductibilité électrique ou respectivement aux propriétés isolantes est relié à ladite zone centrale de l'électrode via un engrènement géométrique.

14. Procédé de fabrication d'une électrode selon une quelconque des revendication 1 à 13, comprenant les étapes suivantes: - d'abord, un corps d'électrode (1) est produit, qui comprend au moins une zone électriquement conductible - ensuite, des cavités (1) sont formées dans cette zone de la surface du corps d'électrode (1), qui vient en contact avec le tissu à traiter - et lesdites cavités (11) sont remplies d'un matériau (12) à basse conductibilité électrique de façon à créer une surface lisse.

15. Procédé selon la revendication 14,
**caractérisé en ce que** lesdites cavités (11) sont remplies par un processus de revêtement.

16. Procédé selon la revendication 15,
**caractérisé en ce que** ledit processus de revêtement est un processus de métallisation sous vide.

17. Procédé selon une quelconque des revendication 14 à 16,
**caractérisé en ce que**, suivant le remplissage desdites cavités (11), au moins cette partie de la surface, qui vient en contact avec le tissu à traiter, est traitée.

18. Procédé selon la revendication 17,
**caractérisé en ce que** ladite opération de traitement est une opération d'usinage.

19. Procédé selon une quelconque des revendication 14 à 18,
**caractérisé en ce que** des contre dépouilles etc. sont formées dans lesdites cavités (11) de façon, que ledit matériau (12) à basse conductibilité électrique s'adhère audit corps d'électrode (1) via un engrènement géométrique.
